# FASCICULE DE BREVET EUROPEEN

(11) **EP 1 617 780 B1**
(45) Date de publication et mention de la délivrance du brevet: **21.06.2017**
(21) Numéro de dépôt: 04742589.7
(22) Date de dépôt: 28.04.2004
(51) Int. Cl.: A61B 17/86, A61B 50/30, A61B 50/00, A61B 17/80

(54) **EMBALLAGE POUR LA PRESENTATION INDIVIDUELLE D'AU MOINS UNE VIS**
VERPACKUNG ZUR BEREITSTELLUNG VON MINDESTENS EINER SCHRAUBE
PACKAGING FOR THE INDIVIDUAL PRESENTATION OF AT LEAST ONE SCREW

(30) Priorité: 28.04.2003 FR 0305150
(43) Date de publication de la demande: 25.01.2006
(73) Titulaire: Caron, Philippe, Papeete 98713, BP 1040 Tahiti (FR)
(72) Inventeur: Caron, Philippe, Papeete 98713, BP 1040 Tahiti (FR)
(74) Mandataire: Cardy, Sophie Marie
(86) Numéro de dépôt international: PCT/FR2004/001022
(87) Numéro de publication internationale: WO 2004/096073

(56) Documents cités:
- WO-A-03/020148
- FR-A- 2 746 629
- US-A- 4 856 648
- US-A- 5 582 299
- US-A- 5 961 330
- US-A- 6 086 371

## Description

L'invention concerne un emballage pour la présentation individuelle d'au moins une vis comprenant une tige et une tête, en particulier une vis d'ostéosynthèse.

Egalement, la présente invention se rapporte à la réalisation d'un ensemble d'emballage et de présentation de matériel d'ostéosynthèse comprenant un emballage de type tel que mentionné précédemment.

Aussi, la présente invention concerne un ensemble de protection comprenant un ensemble d'emballage et de présentation de matériel d'ostéosynthèse de type tel que mentionné précédemment.

Des emballages du type précité sont connus par FR2 746 629 et US5,961,330 (le préambule de la revendication 1 est basé sur ce document).

Les vis et les plaques d'ostéosynthèse permettent d'effectuer une réduction de fracture osseuse, à savoir de remettre et de maintenir en position deux fragments osseux l'un par rapport à l'autre.

De telles vis et plaques d'ostéosynthèse sont notamment classiquement utilisées au cours d'interventions de chirurgie maxillo-faciale, que ce soit à visée réparatrice pour réduire une fracture accidentelle, ou à visée fonctionnelle et/ou esthétique comme dans le cas des ostéotomies de la mandibule et du maxillaire.

Au cours de ces interventions chirurgicales, le chirurgien dispose d'un lot de plaques et d'un lot de vis de fixation qui ont été au préalable sorties de leur emballage respectif (par exemple d'un récipient contenant les plaques et d'un autre récipient contenant les vis de fixation) avant d'être stérilisées puis placées sur un plateau qui reste accessible tout au long de l'intervention.

Selon le type d'intervention à effectuer, le lot de plaques peut comprendre de une à plusieurs plaques, identiques ou non, et le lot de vis peut comprendre de une à plusieurs vis, identiques ou non. Ces lots seront généralement constitués de plus d'une pièce car même dans le cas où une seule pièce est requise pour l'intervention, il est nécessaire de prévoir au moins une autre pièce de rechange au cas où la première pièce manipulée a été abîmée mécaniquement ou bien si son état d'asepsie n'est plus satisfaisant.

En outre, même dans le cas ou le type de plaques et le type de vis à utiliser est préétabli, il peut rester des incertitudes quant aux dimensions les plus adaptées à la morphologie du patient, notamment en ce qui concerne la longueur des vis à utiliser.

En conséquence, de nombreuses manipulations sont nécessaires avant, pendant et après l'intervention chirurgicale en relation avec ces éléments (plaques et vis) formant un lot de matériel d'ostéosynthèse :
- avant l'intervention :le personnel de bloc doit préparer les éléments du lot de matériel d'ostéosynthèse, c'est-à-dire connaître le ou les types de vis et de plaques à prévoir, ainsi que leur nombre, les chercher dans le stock, puis ces éléments sont stérilisés et mis dans une enveloppe étanche ; ces éléments restent ensuite en attente jusque peu de temps avant l'intervention : à ce moment là, on ouvre l'enveloppe étanche et les éléments sont alors placés sur le plateau précité ;
- pendant l'intervention :tout ou partie des éléments du lot de matériel d'ostéosynthèse vont être saisis par une pince ou un autre instrument adapté avant d'être mis en place et fixés sur le patient grâce aux instruments prévus à cet effet ; l'aller-retour de cette pince (ou de cet autre instrument) entre le patient, ou les instruments précités, et le plateau engendre des risques de souillure et de contamination des éléments non encore utilisés restés sur le plateau ; et
- après l'intervention : du fait de ce qui précède, les éléments restants qui ne sont pas détériorés du point de vue mécanique vont devoir de nouveau subir une étape de nettoyage et de stérilisation, qu'ils aient été touchés par un instrument ou non, avant de retourner dans le stock dans l'attente d'être sélectionnés en vue d'une prochaine intervention ; en effet, ces éléments restant ne sont de toute manière plus stériles.

La présente invention a pour objectif de résoudre les problèmes précités afin de permettre une diminution des manipulations à réaliser au bloc opératoire, y compris en relation avec la stérilisation, de façon à fournir au chirurgien un lot de matériel d'ostéosynthèse directement utilisable au bloc opératoire, tout en garantissant la qualité et la stérilité de ce matériel d'ostéosynthèse.

A cet effet, selon un premier objet de la présente invention, est proposé un emballage tel que défini par la revendication 1. Cet emballage comporte un tube apte à être saisi manuellement et présentant une première extrémité et une deuxième extrémité, ledit tube étant pourvu d'au moins un logement destiné à recevoir de manière unitaire ladite vis, ledit logement présentant une ouverture débouchant sur ladite première extrémité, et des moyens de fermeture refermant ledit logement de manière étanche et aptes à être ouverts, ledit logement s'étendant selon une direction longitudinale et présentant une première portion destinée à recevoir ladite tige de la vis et une deuxième portion, ladite deuxième portion étant plus large que la première portion et étant apte à recevoir avec appui ladite tête de la vis, ladite deuxième extrémité servant de socle apte à venir en appui sur un plan sensiblement horizontal, en particulier afin de permettre d'accéder à la vis située dans ledit logement depuis ladite ouverture lorsque lesdits moyens de fermeture sont ouverts, ledit socle étant perpendiculaire à ladite direction longitudinale.

De cette manière, on comprend que par la présence du logement dans le tube, la vis est disposée dans un emplacement stérilisable qui permet en outre un accès direct par la pointe de la lame du tournevis une fois que les moyens de fermeture sont ouverts.

Plus précisément, un tel emballage permet une prise aisée et directe de la vis par le chirurgien qui tient fermement d'une main le tube qu'il a préalablement saisi, le tube étant en appui sur son socle, et de l'autre main il manoeuvre le tournevis pour que la pointe de la lame pénètre dans l'empreinte située sur la tête de la vis, cette dernière restant en place grâce à l'appui de la tête de la vis dans la deuxième portion du logement, la résistance à l'encontre de l'effort d'appui exercé par la pointe étant réalisée par la deuxième portion du logement.

Globalement, grâce à l'agencement selon la présente invention, il est possible de réaliser simultanément l'emballage ainsi que la protection d'un matériel d'ostéosynthèse stérile, et également sa présentation pour une prise directe par l'outil, à savoir le tournevis sans aucune autre manipulation préalable que l'ouverture des moyens de fermeture qui referment l'ouverture du logement du tube. En particulier, il n'est pas nécessaire de sortir la vis du tube avant de la saisir ce qui limite le nombre de manipulations à effectuer et donc cela réduit les risques de contamination avant la pose de cette vis par le chirurgien.

Egalement, selon un deuxième objet de la présente invention, il est prévu un ensemble d'emballage et de présentation pour un lot de matériel d'ostéosynthèse tel que défini par la revendication 9. Cet ensemble d'emballage et de présentation comprenant un ou plusieurs éléments, identiques ou différents, de même nature ou non, dont au moins une vis d'ostéosynthèse logée dans un emballage du type précité selon le premier objet.

En particulier, selon ce deuxième objet, on cherche à obtenir un ensemble pour lequel on garantit visuellement une première utilisation depuis la stérilisation d'un lot de matériel d'ostéosynthèse.

Selon un premier mode de réalisation du deuxième objet, est proposé un ensemble d'emballage et de présentation individuelle d'une série de vis comprenant une tige et une tête, en particulier pour des vis d'ostéosynthèse, caractérisé en ce qu'il comporte au moins deux emballages du type précité selon le premier objet renfermant chacun ladite vis dans le logement et une enveloppe permettant de relier entre eux lesdits emballages en les retenant un à un de manière étanche sous forme d'un chapelet.

Un tel agencement permet de conserver ensemble un lot de vis d'ostéosynthèse en ne prélevant que la ou les vis nécessaire(s) au moment de l'opération chirurgicale, ces vis étant prélevées une à une au niveau de chaque compartiment de l'enveloppe.

Selon un deuxième mode de réalisation du deuxième objet, est proposé un ensemble d'emballage et de présentation individuelle d'une vis comprenant une tige et une tête, en particulier une vis d'ostéosynthèse, caractérisé en ce qu'il comporte un étui de protection et de conditionnement entourant un emballage du type précité selon le premier objet renfermant ladite vis dans le logement.

Selon un troisième mode de réalisation du deuxième objet, est proposé un ensemble d'emballage et de présentation d'un lot de matériel d'ostéosynthèse, ledit lot de matériel d'ostéosynthèse comprenant au moins une plaque et au moins une vis d'ostéosynthèse, caractérisé en ce qu'il comprend une barquette, un film thermoscellable refermant ladite barquette et délimitant avec cette dernière un espace de rangement, ladite plaque étant disposée dans ledit espace de rangement, et un emballage du type précité selon le premier objet renfermant ladite vis dans le logement, ledit emballage étant disposé dans ledit espace de rangement.

Selon un quatrième mode de réalisation du deuxième objet, est proposé un ensemble d'emballage et de présentation d'un lot de matériel d'ostéosynthèse, ledit lot de matériel d'ostéosynthèse comprenant au moins une plaque et au moins une vis d'ostéosynthèse, caractérisé en ce qu'il comprend un support rigide délimitant plusieurs compartiments de rangement parmi lesquels au moins un compartiment de rangement d'un premier type dans lequel est inséré un emballage du type précité selon le premier objet contenant ladite vis et au moins un compartiment de rangement d'un deuxième type destiné à recevoir au moins une plaque d'ostéosynthèse.

Selon une variante du quatrième mode de réalisation du deuxième objet, ledit support rigide forme une partie de fond et il comprend en outre une partie de couvercle, ladite partie de couvercle étant apte à coopérer de manière réversible avec ladite partie de fond entre une position d'ouverture et une position de fermeture, et, dans ladite position de fermeture, ladite partie de couvercle est apte à retenir ledit emballage dans ledit compartiment de rangement du premier type et ladite plaque d'ostéosynthèse dans ledit compartiment de rangement du deuxième type.

Ces différents modes de réalisation permettent de proposer chacun un ensemble d'emballage et de présentation contenant un lot stérile de matériel d'ostéosynthèse de nature différente et/ou répondant à un mode de présentation différent.

Egalement, selon une variante des premier à quatrième modes de réalisation du deuxième objet, on prévoit que l'ensemble d'emballage et de présentation comprend en outre un support d'identification portant des informations visuelles en relation avec ledit lot de matériel d'ostéosynthèse, lesdites informations visuelles comportant un code à barres.

Cette avantageuse disposition permet une traçabilité du lot de matériel d'ostéosynthèse contenu dans l'ensemble d'emballage et de présentation, c'est-à-dire son suivi. En effet, parmi les informations figurant sur le support d'identification, soit sous forme de codes à barres soit sous forme de description écrite ou de dessins ou de plusieurs d'entre eux, on peut prévoir la nature du matériau composant ce matériel, les dimensions de ce matériel, son numéro de série ou de lot afin d'identifier son origine, sa date de stérilisation ainsi que les moyens décontamination et les moyens de stérilisation (en étuve, sous rayon gamma....) et par exemple une date limite conseillée d'utilisation.

Un tel support d'identification est par exemple composé d'une ou de plusieurs étiquettes portant chacune tout ou partie de ces informations, ces étiquettes pouvant se décoller et être recollées ailleurs, par exemple sur le dossier du patient qui va recevoir ou sur lequel a été utilisé le matériel d'ostéosynthèse contenu dans ledit ensemble d'emballage et de présentation.

Ce support d'identification est avantageusement directement positionné sur chaque emballage selon le type du premier objet, c'est-à-dire sur le tube.

Ce support d'identification peut être placé à la fois sur le tube de l'emballage de la vis et sur un autre élément appartenant à l'ensemble d'emballage et de présentation selon le deuxième objet.

Egalement, selon un troisième objet de la présente invention, il est proposé un ensemble de protection tel que défini par la revendication 25. Cet ensemble de protection caractérisé en ce qu'il comporte un ensemble d'emballage et de présentation du type précité selon le deuxième objet, une barquette de protection formant un contenant pour ledit ensemble d'emballage et de présentation et un film thermoscellable apte à être retiré manuellement refermant ladite barquette de manière étanche. De cette manière, on propose une protection renforcée du lot de matériel d'ostéosynthèse aux différentes contraintes auxquelles il peut être soumis jusqu'à son utilisation.

D'autres avantages et caractéristiques de l'invention ressortiront à la lecture de la description suivante de plusieurs modes de réalisation de l'invention faite à titre d'exemple et en référence aux dessins annexés dans lesquels :
- la figure 1 montre une vue en perspective éclatée d'une première variante d'un emballage,
- la figure 2 montre une vue en perspective éclatée d'une deuxième variante d'un emballage,
- la figure 2A est une coupe longitudinale de l'emballage visible à la figure 2,
- la figure 3 montre une vue en perspective partielle d'une troisième variante de l'emballage de la figure 1,
- la figure 3A est une coupe longitudinale partielle de la partie de l'emballage visible à la figure 3,
- les figures 4A et 4B montrent une vue en coupe longitudinale de deux alternatives d'une variante de l'emballage selon le premier objet de la présente invention,
- la figure 5 montre l'utilisation de l'emballage de la figure 4 dans une première étape,
- la figure 6 montre l'utilisation de l'emballage de la figure 4 dans une deuxième étape,
- la figure 7 représente schématiquement un premier mode de réalisation de l'ensemble d'emballage et de présentation formant le deuxième objet de la présente invention,
- la figure 8 représente schématiquement un deuxième mode de réalisation de l'ensemble d'emballage et de présentation formant le deuxième objet de la présente invention,
- la figure 9 représente un troisième mode de réalisation de l'ensemble d'emballage et de présentation formant le deuxième objet de la présente invention,
- les figures 10 à 13 représentent en perspective plusieurs variantes d'un quatrième mode de réalisation de l'ensemble d'emballage et de présentation formant le deuxième objet de la présente invention,
- la figure 14 est une vue en perspective éclatée d'un ensemble de protection formant le troisième objet de la présente invention et constitué d'une barquette de protection et de l'ensemble d'emballage et de présentation formant le deuxième objet de la présente invention, et
- la figure 15 est une vue en perspective depuis l'un des coins et depuis le dessus d'une variante d'un ensemble de protection formant le troisième objet de la présente invention et présentant un étui de protection, dans sa position d'ouverture, contenant la barquette de la figure 14.

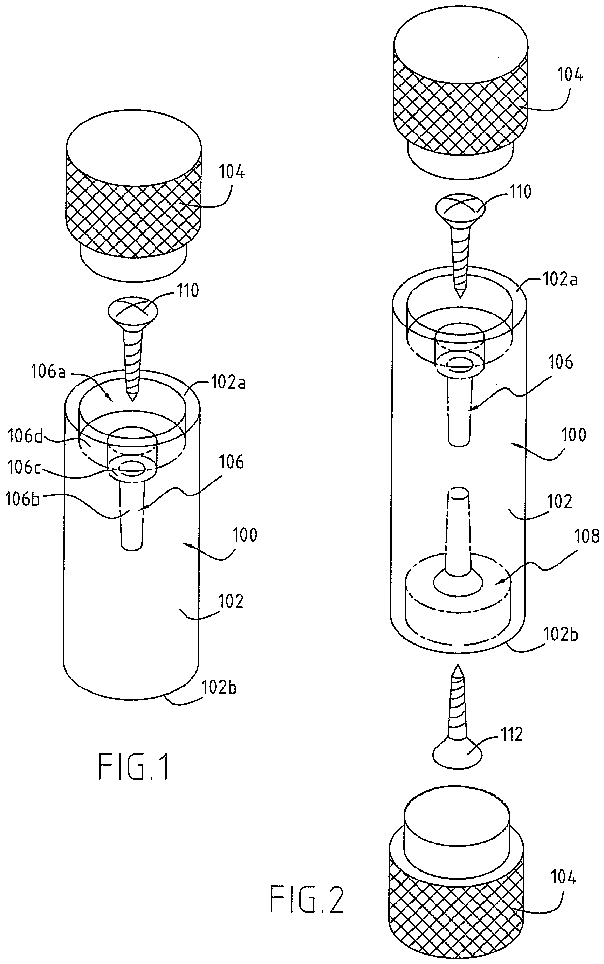

On se reporte à la figure 1 sur laquelle est visible, sous forme éclatée, une première variante d'un emballage 100 composé d'un tube 102, d'un bouchon 104 et d'une vis 110.

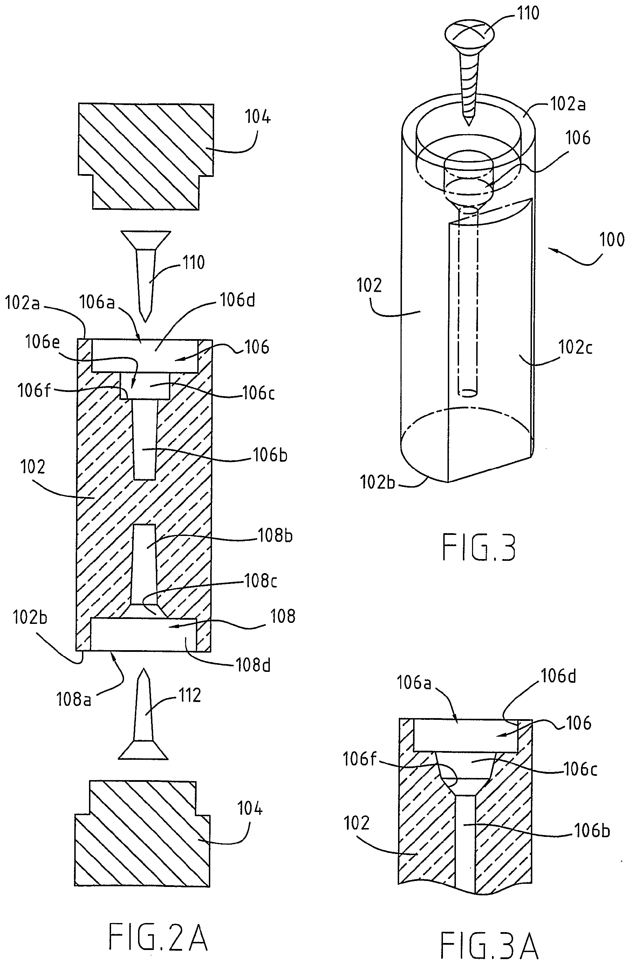

Le tube 102 est représenté comme un cylindre de section circulaire mais il est entendu que le tube peut être cylindrique ou non, avec toute autre forme de section convenant pourvu que le tube reste facilement préhensible entre le pouce et l'index d'une même main. Le tube 102 s'étend entre une première extrémité 102a (en haut sur la figure 1) et une deuxième extrémité 102b (en bas sur la figure 1). La deuxième extrémité 102b est délimité par une face plane orthogonale à la direction principale du tube 102, cette face plane servant de socle lorsque le tube est posé debout sur une surface sensiblement plane. Un logement 106 (voir le haut de la figure 2A) débouche sur la première extrémité 102a à l'emplacement d'une ouverture 106a. ce logement 106 présente une symétrie de révolution. Ce logement comporte une première portion 106b tronconique allant jusqu'au fond du logement et destinée à recevoir la tige filetée de la vis 110, cette première portion pouvant également présenter une autre forme, par exemple cylindrique.

Cette première portion 106b se prolonge en direction de l'ouverture 106a par une deuxième portion 106c plus large destinée à recevoir la tête de la vis 110 et qui est ici présente sous la forme d'un cylindre de section circulaire. On peut aussi prévoir une deuxième portion 106c ayant la forme d'un tronc de cône allant en se rétrécissant en direction de la première portion, selon un angle assez faible (5 à 45°) par rapport à la direction verticale (direction principale du tube 102). Cette forme en tronc de cône de la deuxième portion 106b forme une paroi inclinée facilitant le centrage de la lame du tournevis en direction du centre de la tête de la vis, c'est-à-dire de l'axe du tube 102.

Cette deuxième portion 106c se prolonge en direction de l'ouverture 106a par une troisième portion 106d plus large destinée à recevoir des moyens de fermeture étanches formés par un bouchon 104. Sur la figure 1, la troisième portion 106d est présente sous la forme d'un cylindre de section circulaire.

En particulier, il est prévu qu'entre ladite deuxième portion 106c et ladite première portion 106b est formé un épaulement 106e muni d'une face d'appui sensiblement plane 106f dirigée en direction de ladite ouverture 106a , ladite tête de la vis venant en appui sur ladite face d'appui sensiblement plane 106f. Sur les figures 1, 2 et 2A, la face d'appui 106f est perpendiculaire à la direction longitudinale du logement 106, c'est-à-dire qu'elle est horizontale lorsque le tube 102 est debout. D'autres formes sont possibles pour cette face d'appui 106f pourvu qu'elle puisse recevoir la face de la tête de la vis tournée vers la tige de la vis. En particulier, on choisit, pour cette face d'appui 106f, une forme complémentaire à celle de la face de la tête de la vis tournée vers la tige de la vis, en particulier une forme tronconique.

La notion d'étanchéité, qui est réalisé par la portion du bouchon 104 qui coopère de manière serrée avec la face interne de la troisième portion 106d du logement 106, est définie comme un rempart à la pénétration de fluide, à savoir un gaz ou un liquide. Cette étanchéité est brisée après la première ouverture du bouchon 104 qui forme des moyens de fermeture, ce qui ne permet alors plus de garantir le maintien de la stérilité à l'intérieur du logement 106.

Ainsi, on comprend que lesdits moyens de fermeture comprennent un bouchon 104 et que ledit logement 106 présente une troisième portion 106d plus large que la deuxième portion 106c qui lui est adjacente, ladite troisième portion 106d étant apte à recevoir ledit bouchon 104 de manière serrée de manière à refermer ledit logement 106 de manière réversible.

De préférence, le tube 102 est cylindrique de section transversale au moins partiellement circulaire, pour faciliter sa prise en main par le pouce et l'index entre sa première et sa deuxième extrémités 102a et 102b. Sur la variante de la figure 1, la section transversale du tube 102 est circulaire. D'autre part, la première portion 106b et la deuxième portion 106c du logement 106 présentent également une forme de cylindre de section circulaire mais d'autres formes sont possibles pourvu qu'elles permettent le passage de la tête de la vis 110 et que la troisième portion 106d présente une forme complémentaire de celle de la face extérieure de la partie inférieure du bouchon 104.

Dans le cas de la première variante de réalisation de l'emballage de la figure 1, ledit tube 102 comporte un seul logement 106 débouchant à la première extrémité 102a.

La deuxième variante de réalisation d'un emballage est illustrée sur les figures 2 et 2A est similaire à l'emballage décrit précédemment en relation avec la figure 1 sauf sur les points qui suivent. Le tube 102 de la figure 2 comporte deux logements, à savoir un premier logement 106 tel que celui de la figure 1 et qui débouche à la première extrémité 102a du tube 102 et le tube 102 comporte également un second logement 108 destiné à recevoir de manière unitaire une seconde vis 112.

Ledit second logement 108 présente une ouverture 108a débouchant sur ladite deuxième extrémité 102b. Des seconds moyens de fermeture (formés d'un bouchon non représenté) referment ledit second logement 108 de manière étanche et sont aptes à être ouverts. Ledit second logement 108 s'étend selon une direction longitudinale et présente une première portion 108b destinée à recevoir la tige de la seconde vis 112 et une deuxième portion 108c, ladite deuxième portion 108c étant plus large que la première portion 108b et étant apte à recevoir avec appui ladite tête de la seconde vis 112.

En outre, afin de retirer la seconde vis 112 située dans le second logement 108, l'emballage 100 de la figure 2 étant retourné de 180°, ladite première extrémité 102a sert alors de socle apte à venir en appui, éventuellement avec interposition du bouchon 104, sur un plan sensiblement horizontal, en particulier afin de permettre d'accéder à la vis 112 située dans ledit second logement 108 depuis ladite ouverture 108a dudit second logement 108 lorsque lesdits seconds moyens de fermeture sont ouverts, ledit socle étant perpendiculaire à ladite direction longitudinale. La première portion 108b destinée à recevoir la tige de la seconde vis 112 s'évase en direction de ladite ouverture 108a. En outre, la deuxième portion 108c présente au moins une première partie en forme de tronc de cône, ladite tête de la seconde vis venant en appui sur ladite forme de tronc de cône. De façon particulière et tel que représenté sur les figures 2 et 2A, toute la deuxième portion 108c du second logement 108 présente une forme de tronc de cône. On peut prévoir que la première portion 108b de ce second logement 108 est plus courte que la première portion 106b du premier logement 106 dans le cas où le second logement 108 est destiné à recevoir une vis 112 plus courte que la vis 110 logé dans le premier logement 106.

Lors du conditionnement des vis 110 et 112 dans le tube 102, les vis 110 et 112, préalablement décontaminées, sont placées dans leur logement 106 et 108 respectif, les bouchons 104 correspondant sont placées dans la troisième portion 106d et 108d des logements 106 et 108 puis l'ensemble est stérilisé, de préférence à froid et sous rayon gamma. Ainsi, les vis 110 et 112 sont stériles jusqu'au retrait du bouchon 104.

La troisième variante de réalisation de l'emballage de la figure 3 est similaire à celui décrit précédemment en relation avec l'emballage de la figure 1 sauf sur les points qui suivent. Le tube 102 présente une section de cercle en partie haute contenant la première extrémité 102a. En partie basse et au milieu , la section du tube 102 est un cercle tronqué par une ligne droite de façon à définir une face latérale plane ou méplat 102c pouvant faciliter le repérage de la position du tube ainsi que sa prise en main. La deuxième portion 106c du logement comporte dans ce cas une première partie adjacente à la première portion 106b qui est de forme tronconique et une deuxième partie de forme tronconique adjacente à la troisième portion 106d. On comprend que la tête de la vis peut venir en appui sur la première partie tronconique de la deuxième portion 106c qui forme un angle plus grand que la deuxième partie de la deuxième portion 106c par rapport à l'axe principal ou la direction longitudinale (verticale sur la figure 3) du tube 102.

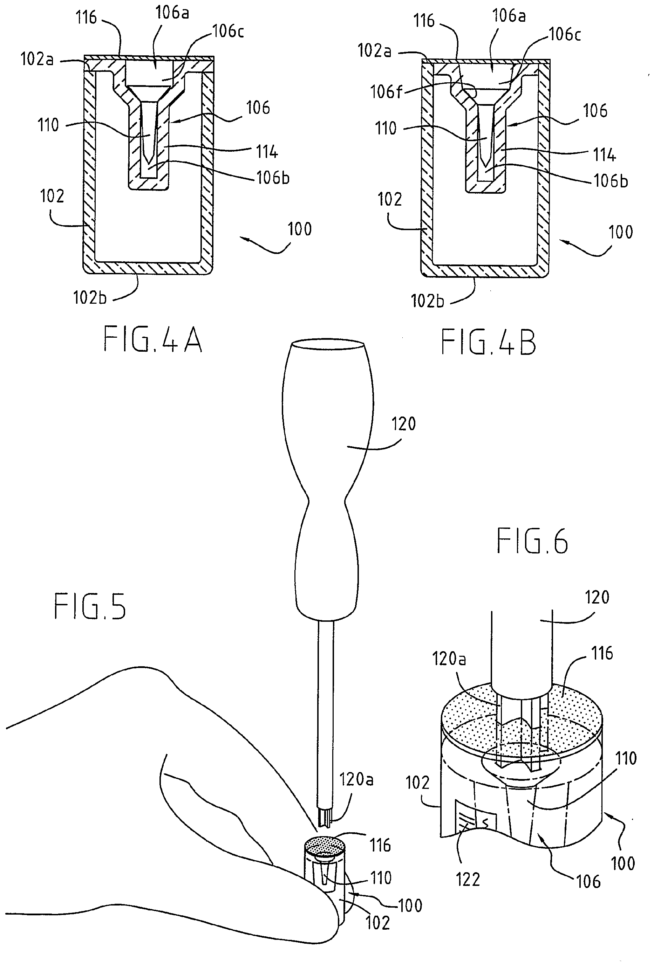

La quatrième variante de réalisation de l'emballage des figures 4A et 4B, qui forme le premier objet selon la présente invention, est similaire à celui décrit précédemment en relation avec l'emballage de la figure 1 sauf sur les points qui suivent. En particulier, au contraire du tube 102 des variantes citées précédemment en relation avec les figures 1 à 3, qui est un tube plein sauf à l'emplacement du (ou des) logement(s) 106 (et 108), le tube 102 des figures 4A et 4B est creux et le logement 106 est formé dans une pièce tubulaire 114 disposée au moins partiellement à l'intérieur dudit tube 102, un espace fermé étant formé entre le tube 102 et la pièce tubulaire 114.

En particulier, pour l'alternative représentée sur la figure 4A, la pièce tubulaire 114 est pratiquement entièrement logée dans le tube 102, l'ouverture 106a du logement 106 délimité par cette pièce tubulaire 114 étant située presque à la même hauteur et en fait à peine un peu plus haut que la première extrémité 102a du tube 102.

Dans le cas de l'alternative représentée sur la figure 4B, la pièce tubulaire 114 est ici entièrement logée dans le tube 102, l'ouverture 106a du logement 106 délimité par cette pièce tubulaire 114 étant située exactement à la même hauteur que la première extrémité 102a du tube 102 car la première extrémité 102a du tube 102 est alignée avec la face supérieure de la deuxième portion 106c du logement qui délimite l'ouverture 106a.

Dans les deux cas (figures 4A et 4B), la pièce tubulaire 114 et le tube 102 sont reliés ensemble le long de leurs surfaces en contact, par exemple par soudage par ultrasons.

Comme alternative (non représentée), la pièce tubulaire 114 et le tube 102 sont obtenus par une étape d'injection unique, seule la deuxième extrémité 102b (formant ici un fond) est rapportée séparément: dans ce cas la paroi latérale et la première extrémité 102a du tube 102, ainsi que la pièce tubulaire 114 sont réalisées en une seule pièce.

Egalement, il faut noter que le logement 106 défini par la pièce tubulaire 114 ne comporte pas de troisième portion mais seulement une première portion 106b (de forme cylindrique légèrement tronconique et effilée vers le fond) pour loger la tige d'une vis et une deuxième portion 106c pour loger la tête de cette vis. Cette deuxième portion présente la même forme que la deuxième portion 106c du logement 106 de la troisième variante de réalisation illustrée sur les figures 3 et 3A, à savoir une première partie tronconique avec la face d'appui 106f très évasée et une deuxième partie tronconique peu évasée, adjacente à l'ouverture 106a.

Enfin, il faut noter que les moyens de fermeture sont constitués par un opercule 116 formé d'un film plastique thermoscellable qui a la particularité de pouvoir être facilement retiré et, éventuellement, de pouvoir être perforé par la pointe 120a d'un tournevis 120 (voir figures 5 et 6) pour accéder à la tête de la vis 110 dont la face inférieure est en appui sur la portion de paroi en forme de tronc de cône formant la première partie de la deuxième portion du logement 106. Cette étape de perforation et de mise en prise de la pointe 120a du tournevis avec l'empreinte de la tête de la vis 110 est illustrée sur la figure 6.

Dans ce cas, on comprend que lesdits moyens de fermeture comprennent un opercule 116, de préférence perforable, qui recouvre l'ouverture 106a du logement 106. il faut noter que cet opercule 116 est donc préalablement relié à l'extrémité supérieure de la pièce tubulaire 114 de façon à refermer le logement 106 de manière étanche. Cette liaison peut avantageusement être réalisée par soudage aux ultrasons et, en particulier dans le cas de l'alternative de la figure 4B, on peut souder en même temps la pièce tubulaire 114, le tube 102 et l'opercule 116.

Dans la première étape de l'utilisation de l'emballage 100 de la figure 4A ou 4B, telle qu'illustrée sur la figure 5, le chirurgien tient fermement d'une main, entre le pouce et l'index, le tube 102 qu'il a préalablement saisi, le tube étant en appui par son socle formé de la deuxième extrémité 102b (en bas sur la figure 5) sur un support tel qu'une table. Ensuite, dans une deuxième étape de l'utilisation de l'emballage de la figure 4, de l'autre main le chirurgien manoeuvre le tournevis 120 pour que la pointe 120a de la lame perfore l'opercule 116, se dirige vers (grâce à la forme un peu évasée de la deuxième partie de la deuxième portion 106c), puis pénètre dans l'empreinte située sur la tête de la vis qui reste bien en place dans son logement grâce à l'appui de la tête de la vis dans la première partie de la deuxième portion 106c du logement (face d'appui 106f), la résistance à l'encontre de l'effort d'appui exercé par la pointe étant réalisée par la première partie de la deuxième portion du logement 106. Ensuite, dans une troisième étape non représentée, le chirurgien sort la vis 110 de son logement 106 en écartant simplement le tournevis 120 de l'emballage 100. Ainsi, aucun contact n'a eu lieu entre la vis et un élément extérieur à part la pointe de la lame du tournevis, ce qui limite les manipulations (gain de temps et de précision) et les risques de contamination de la vis qui reste ainsi stérile hors de l'emballage 100.

Pour faciliter la prise de la vis et éviter toute interférence avec l'opercule 116, lors de la deuxième étape précitée, il est possible de décomposer le mouvement en réalisant la perforation et/ou la déchirure de l'opercule 116, puis d'effectuer une rotation la pointe 120a de la lame du tournevis pour agrandir le passage dans l'opercule 116, avant d'introduire la pointe 120a plus avant dans le logement 106 pour atteindre la tête de la vis 110.

Egalement, de préférence, il est prévu que ledit tube 102 porte au moins un support d'identification 122 portant des informations visuelles en relation avec ladite vis 110 contenu dans le logement 106, lesdites informations visuelles comportant un code à barres. Sur la figure 6, apparaît un tel support d'identification 122 formé d'une étiquette qui peut avantageusement se décoller pour être recollée dans le dossier du patient qui va recevoir la vis 110 en question.

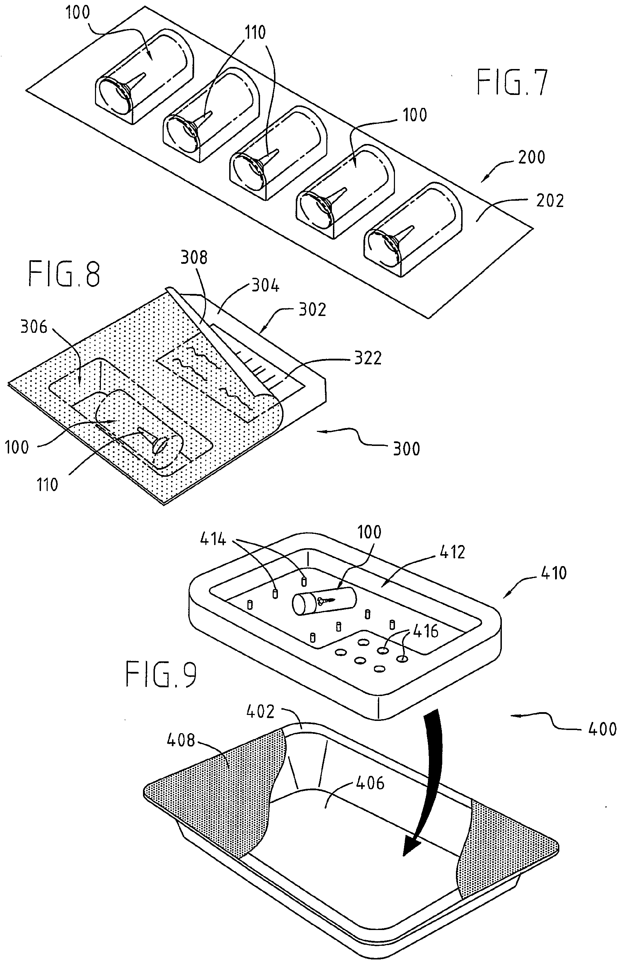

Le deuxième objet de la présente invention va maintenant être décrit en relation avec les figures 7 à 13 représentant plusieurs modes de réalisation.

Selon le premier mode de réalisation du deuxième objet illustré sur la figure 7, il est prévu de placer de manière séparée plusieurs emballages 100 conformes au premier objet décrit précédemment, dans une enveloppe plastique 202 délimitant un compartiment fermé pour chaque emballage, le tout constituant un ensemble 200 d'emballage et de présentation individuelle d'une série de vis 110. Cet ensemble 200 peut être stérilisé aux rayons gamma et proposé au bloc opératoire pour permettre d'avoir à tout moment, tant que tous les compartiments ne sont pas ouverts, la possibilité de prendre une à une chaque vis 110.

Selon le deuxième mode de réalisation du deuxième objet illustré sur la figure 8, est prévu un ensemble 300 d'emballage et de présentation individuelle d'une vis 110 d'ostéosynthèse. A cet effet, l'ensemble 300 comporte un étui de protection et de conditionnement 302.

Cet étui de protection et de conditionnement 302 est réalisé en matière plastique sous forme d'une partie rigide 304, délimitant un espace 306 renfermant un emballage 100, et d'une partie souple 308 formée d'un film thermoscellable refermant de manière étanche ledit espace 306 et apte à être retiré manuellement.

A la place ou en plus d'une étiquette 122 placée sur la face externe du tube 102 de l'emballage 100 (voir figure 6), on prévoit un autre support d'identification 322 portant des informations en relation avec ledit emballage 100 et la vis 110 qu'il contient, notamment sous la forme d'un code à barres. Sur la figure 8, cet autre support d'identification 322 est collé sur un emplacement disposé sur la face extérieure de la partie rigide 304 de l'étui de protection et de conditionnement 302.

Cet ensemble 300 peut être conditionné dans une boîte en carton avec d'autres ensembles 300 identiques placés les uns derrière les autres.

On se reportera maintenant à la figure 9 illustrant le troisième mode de réalisation du deuxième objet selon la présente invention consistant en un ensemble 400 d'emballage et de présentation d'un lot de matériels d'ostéosynthèse comprenant au moins une plaque et au moins une vis d'ostéosynthèse. Cet ensemble 400 comprend une barquette 402 (en plastique, aluminium ou tout autre matériau), un film thermoscellable 408 refermant la barquette 402 de manière étanche et délimitant avec cette dernière un espace de rangement 406 dans lequel une ou plusieurs plaques d'ostéosynthèse (non représentée(s)) peu(ven)t être disposée(s). Dans cet espace de rangement 406 est également disposé au moins un emballage 100 individuel d'une vis correspondant au premier objet de la présente invention.

Avantageusement, cet ensemble 400 comprend en outre au moins un insert placé dans l'espace de rangement 406. Dans le cas de la variante illustrée sur la figure 9, un seul insert 410 est disposé dans l'espace de rangement 406 en recouvrant tout le fond de la barquette 402, cet insert en matière plastique étant muni d'au moins une cavité 412 destinée à recevoir au moins une plaque d'ostéosynthèse. L'insert 410 représenté sur la figure 9 comprend une grande cavité 412 en forme de L apte à recevoir côte à côte plusieurs plaques d'ostéosynthèse ainsi qu'un ou plusieurs emballages 100. Cette cavité 412 est munie de moyens de retenue permettant le positionnement de la ou les plaques d'ostéosynthèse. Plus précisément, ces moyens de retenue sont constitués par des picots 414 sur lesquels peuvent être montés les alésages des plaques d'ostéosynthèse.

Egalement, de manière avantageuse il est prévu que l'insert 410 est en outre équipé d'au moins un alvéole 416 destiné à recevoir au moins une vis d'ostéosynthèse. Sur la figure 9, l'insert 410 comporte six alvéoles 416 formant chacun une empreinte creuse dont la forme correspond sensiblement à la forme extérieure d'une vis d'ostéosynthèse. Ces alvéoles 416 sont particulièrement adaptés pour des vis de petite longueur tandis que l'on pourra placer dans l'emballage 100 une vis de plus grande longueur. Ces aménagements peuvent donc être adaptés en fonction du nombre de plaques et de vis et de leurs dimensions afin de fournir dans l'ensemble 400 un lot de matériels d'ostéosynthèse formant un kit complet pour une intervention chirurgicale donnée.

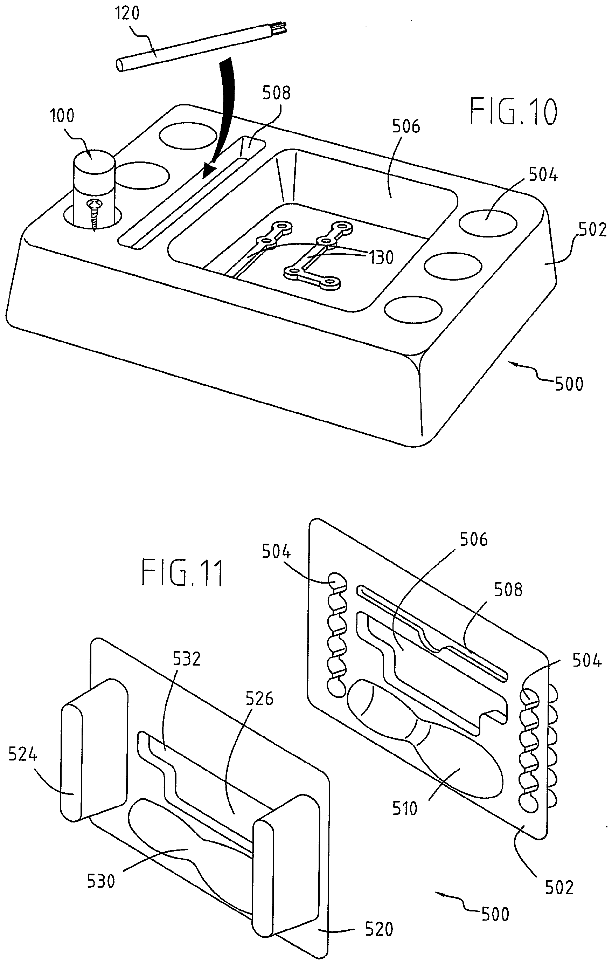

On se reportera maintenant aux figures 10 à 13 montrant plusieurs alternatives de réalisation d'un quatrième mode de réalisation du deuxième objet selon la présente invention formé d'un ensemble 500 d'emballage et de présentation d'un lot de matériels d'ostéosynthèse comprenant au moins une plaque et au moins une vis d'ostéosynthèse. Tous ces ensembles 500 comportent un support rigide 502 réalisé par exemple en matière plastique moulé ou injecté, délimitant plusieurs compartiments de rangements parmi lesquels au moins un compartiment de rangement d'un premier type 504 dans lequel est inséré un emballage 100 du type du premier objet selon la présente invention.

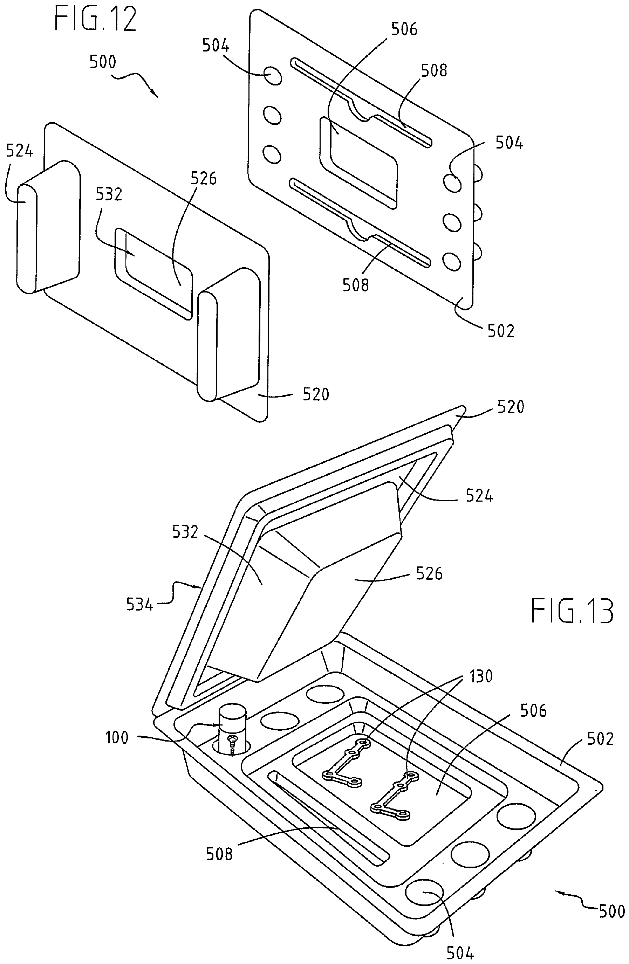

Egalement, parmi les compartiments de rangement du support rigide 502, on trouve également au moins un compartiment de rangement d'un deuxième type 506 destiné à recevoir au moins une plaque d'ostéosynthèse 130 (voir figures 10 et 13). Le support rigide 502 comprend avantageusement également au moins un compartiment de rangement d'un troisième type 508 dans lequel est logé un outil d'ostéosynthèse.

De préférence, le support rigide 502 comprend au moins deux compartiments du premier type 504 recevant chacun un emballage 100 afin que l'ensemble 500 propose la distribution d'au moins deux vis d'ostéosynthèse logées chacune dans un emballage 100.

Dans le cas de l'alternative de réalisation du quatrième mode de réalisation du second objet tel qu'illustré à la figure 10, le support rigide 502 comporte six compartiments de rangement du premier type 504 destinés à recevoir chacun un emballage 100 de sorte que le support rigide 502 peut recevoir six vis. En outre, le support rigide 502 de la figure 10 comporte un compartiment de rangement du deuxième type 506 pouvant recevoir plusieurs plaques d'ostéosynthèse 130, ainsi qu'un compartiment de rangement du troisième type 508 de forme allongée pouvant recevoir notamment un foret utilisé par le chirurgien dans une phase initiale préalable au positionnement et à l'insertion de la vis contenue dans l'emballage 100. Sur la figure 10, le compartiment de rangement du troisième type 508 reçoit au moins une lame 120 d'un tournevis avec mandrin, cette lame ayant sa pointe adaptée à la forme de l'empreinte de la tête des vis 110 des emballages 100.

Sur la figure 11 est illustrée une autre variante de réalisation d'un ensemble 500 selon le quatrième mode de réalisation du second objet de la présente invention qui comporte un support rigide 502 relativement plat et allongé muni de douze compartiments de rangement du premier type 504, d'un compartiment de rangement du deuxième type 506 apte à recevoir plusieurs plaques d'ostéosynthèse, d'un compartiment de rangement du troisième type 508 pouvant lui également recevoir un foret et/ou une lame de tournevis. Egalement, le support rigide 502 de l'ensemble 500 de la figure 11 comprend en outre au moins un compartiment de rangement d'un quatrième type 510 et au moins une portion d'un tournevis, disposée dans le compartiment de rangement du quatrième type 510 du support rigide 502. En fait, comme on peut le voir sur la figure 11, le support rigide 502 de l'ensemble 500 comporte un seul compartiment de rangement du quatrième type 510 apte à recevoir tout ou partie d'un tournevis (non représenté).

En conséquence, on peut prévoir que le support rigide 502 comprend, outre les compartiments de rangement du premier type 504 et du deuxième type 506, au moins un compartiment de rangement d'un troisième type 508 et au moins un foret et/ou une lame de tournevis adapté(e)(s) à la vis contenue dans l'un des emballages 100, et disposé(e)(s) dans le compartiment de rangement du troisième type 508 du support rigide 502.

Avantageusement, tel qu'on peut le voir sur la figure 11, le compartiment de rangement du quatrième type 510 est constitué par une cavité formée de deux lobes correspondant à la forme extérieure d'un tournevis qui comporte une partie de préhension munie d'un tronçon arrière destiné à venir se placer dans le creux de la main et d'un tronçon avant monté rotatif par rapport au tronçon arrière et apte à porter la lame du tournevis, la manipulation du tronçon avant étant réalisée par l'extrémité des doigts de la main retenant dans son creux le tronçon arrière. Ce type de tournevis étant connu, il ne sera pas décrit ni illustré plus avant.

Sur la figure 12 est visible une autre alternative de réalisation du quatrième mode de réalisation du second objet formé d'un ensemble 500 doté d'un support rigide 502 muni de six compartiments de rangement du premier type 504 logeant chacun un emballage 100, un compartiment de rangement du deuxième type 506 apte à loger plusieurs plaques d'ostéosynthèse 130, ainsi que deux compartiments de rangement du troisième type 508 aptes à loger chacun un ou plusieurs forets et/ou lames de tournevis. Dans cette alternative du quatrième mode de réalisation du second objet selon la présente invention représentée sur la figure 12, il n'y a donc aucun compartiment de rangement du quatrième type 510.

Sur la figure 13, est représentée une autre alternative sous la forme d'un ensemble 500 similaire à celui de la figure 12 sauf en ce qu'il ne comporte qu'un seul compartiment de rangement du troisième type 508.

Selon une disposition avantageuse et préférentielle mais non obligatoire, comme illustré sur les figures 11 à 13 le support rigide 502 forme en fait une partie de fond et l'ensemble 500 comprend en outre une partie de couvercle 520 apte à coopérer de manière réversible avec la partie de fond 502 entre une position d'ouverture et une position de fermeture. De cette manière, dans la position de fermeture, la partie de couvercle 520 est apte à retenir chacun des emballages 100 dans son compartiment de rangement du premier type 504 respectif et à retenir la (ou les) plaque(s) d'ostéosynthèse dans son (leur) compartiment de rangement du deuxième type 506. Dans le cas des variantes des figures 11 et 12, la partie de couvercle 520 est une pièce séparée du support rigide 502 tandis que dans le cas de la variante de réalisation de la figure 13, la partie de couvercle 520 est solidaire du support rigide 502 en formant avec cette dernière une charnière pivotante située latéralement sur l'un des bords du support rigide 502 et de la partie de couvercle 520.

Dans le cas de la partie de couvercle 520 de la variante de réalisation de la figure 11, cette dernière comporte deux portions d'un premier type 524 formée chacune d'une zone en creux positionnée en regard chacune d'une série de compartiments de rangement du premier type 504 afin de loger la partie d'un emballage 100 qui fait saillie en dehors d'un compartiment de rangement du premier type 504. Egalement cette partie de couvercle 520 de la figure 11 comporte une portion d'un deuxième type 526 formant une saillie en direction du compartiment de rangement du deuxième type 506 afin de bloquer les plaques dans le compartiment de rangement du deuxième type 506. Egalement, la partie de couvercle 520 de la figure 11 comporte une portion d'un quatrième type 530 présentant une forme similaire à celle du compartiment de rangement du quatrième type 510 de cette figure 11 à savoir conformée selon la forme extérieure du tournevis destiné à être logé et retenu à cet endroit. De préférence, le support rigide 502 et la partie de couvercle 520 sont réalisés en matière plastique et leurs bords comportent des formes complémentaires permettant le clipsage entre ces deux pièces.

Dans le cas de la variante de la figure 12 où la partie de couvercle 520 est également séparée du support rigide 502 formant la partie de fond, la partie de couvercle 520 comporte également deux portions du premier type 524 aptes à recevoir la partie saillante d'une rangée d'emballages 100 logés individuellement dans un compartiment de rangement du premier type 504 au niveau de la partie de fond formée du support rigide 502. La partie de couvercle de la variante de la figure 12 comporte également une portion du deuxième type 526 formée d'une partie allant en saillie du côté et en regard du compartiment de rangement du deuxième type 506 afin de retenir les plaques 130. Dans ce cas, le bord de la partie de fond 502 et de la partie de couvercle 520 ne présentent aucune disposition particulière pour retenir entre eux ces deux parties qui restent positionnées l'une par rapport à l'autre grâce à l'ajustement serré de la portion du premier type 524 autour des emballages 100.

Dans le cas de la variante de la figure 13, la partie de couvercle 520 présente une bordure dont la forme coopère par complémentarité de forme avec la bordure de la partie de fond formée du support rigide 502. Egalement la partie de couvercle 520 de la figure 13 présente, sur sa face dirigée vers la partie de fond 502, une rainure 524 (formant une portion du premier type) sensiblement périphérique et venant en regard des compartiments de rangement du premier type 504 afin de pouvoir recevoir la partie saillante des emballages 100 logés individuellement dans ces compartiments de rangement du premier type 504. Là encore, dans la variante de réalisation de la figure 13, la partie de couvercle 520 comporte une portion du deuxième type 526 formée d'une partie saillante en direction du compartiment de rangement du deuxième type 506 afin de retenir et de bloquer les plaques 130 dans ce compartiment de rangement du deuxième type 506.

Selon une avantageuse disposition, la partie de couvercle 520 présente, sur sa face opposée à la partie de fond 502, un espace de rangement 532 contenant une plaque fantôme et refermée de manière étanche par un fil thermoscellable 534 apte à être retiré manuellement.

Plus précisément, comme on peut le voir sur les figures 11 à 13, la portion du deuxième type 526 de la partie de couvercle qui fait saillie en direction de la partie de fond 502 présente sur sa face arrière une forme en creux délimitant l'espace de rangement 532. De cette manière, on peut loger une plaque fantôme, à savoir une plaque d'ostéosynthèse qui n'est pas réalisée dans le matériau biocompatible de la plaque d'ostéosynthèse qui est réellement posée mais par exemple dans un alliage d'aluminium lui rendant une grande souplesse et permettant au chirurgien de déformer manuellement cette plaque fantôme afin de faire un essai préalable de positionnement au niveau du site d'intervention chirurgicale afin de vérifier qu'une telle forme de plaque avec de telles dimensions est effectivement adaptée à l'intervention qu'il doit réaliser.

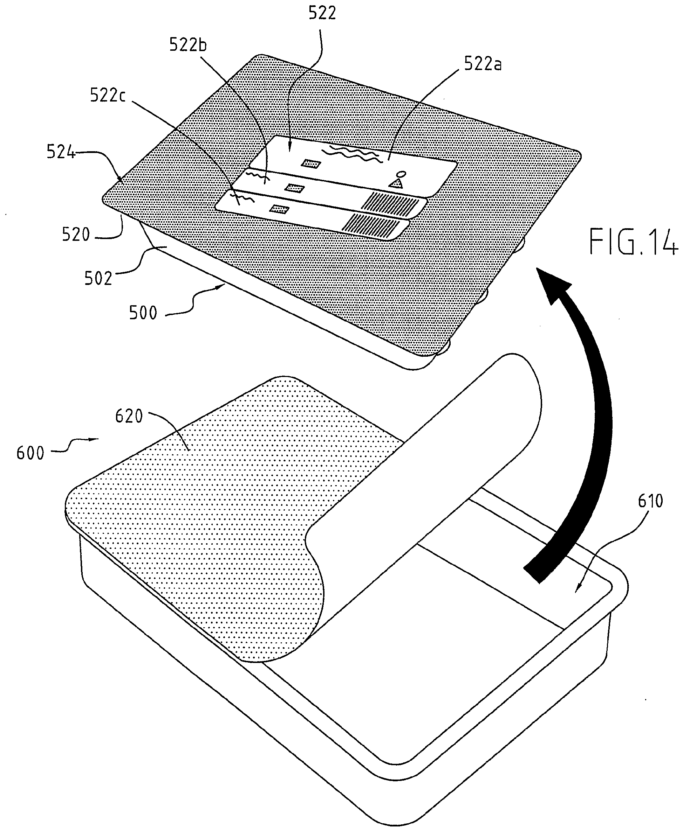

Selon un autre aspect de la présente invention, comme il apparaît sur la figure 14, la face de la partie de couvercle 520 tournée en direction opposée auxdits compartiments de rangement 504, 506 et 508 est munie d'une étiquette 522.

Cette étiquette 522 forme un support d'identification portant des informations en relation avec le lot de matériel d'ostéosynthèse (vis 110 et plaque 130), en particulier sous la forme d'un code à barres. Cette étiquette 522 peut bien entendu être placée ailleurs sur la face extérieure de l'ensemble 500 d'emballage et de présentation.

Plus précisément, l'étiquette 522 comporte trois portions 522a, 522b et 522c, chacune d'ente elles portant la référence de la société qui aura préparé et stérilisé le lot de matériel d'ostéosynthèse situé dans l'ensemble 500 d'emballage et de présentation.

Ces portions 522a, 522b et 522c comportent toute une série d'informations parmi lesquelles le numéro de série de l'ensemble 500 d'emballage et de présentation, le numéro de lot et la référence de chacun des éléments du lot de matériel d'ostéosynthèse (vis 110 et plaque 130), des informations en relation avec la position de chacun des éléments du lot de matériel d'ostéosynthèse parmi les différents logements (compartiments de rangement du premier type 504, du deuxième type 506 ou du troisième type 508).

Ainsi, après l'utilisation de certains des éléments composant le lot de matériel d'ostéosynthèse qui est logé dans l'ensemble 500 d'emballage et de présentation, il est alors possible de connaître les éléments manquants pour réapprovisionner cet ensemble 500 d'emballage et de présentation.

En outre, les portions 522a, 522b et 522c de l'étiquette 522 portant les mêmes informations, la portion 522a va être gardée pour archivage, tandis que la portion 522b sera collée sur le dossier du patient et la portion 522c pourra être utilisée pour le compte rendu de l'intervention chirurgicale.

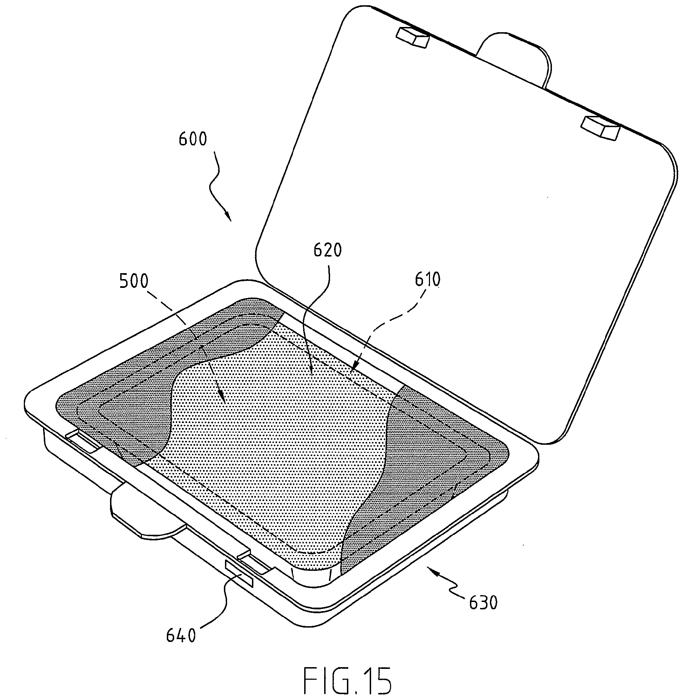

Afin de protéger l'ensemble 500 d'emballage et de présentation durant son transport et avant l'utilisation de l'ensemble d'ostéosynthèse qu'il comporte, la présente invention porte également, selon un troisième objet, sur un ensemble de protection 600 qui comporte, outre l'ensemble 500 d'emballage et de présentation tel que décrit précédemment, également :
- une barquette 610 (voir figure 14) réalisée de préférence en matière plastique transparente ou translucide, cette barquette 610 délimitant un logement apte à recevoir l'ensemble 500 comme il apparaît sur la figure 14. Cette barquette 610 de forme générale rectangulaire se compose également d'une paroi de fond et d'une paroi latérale continue et prolongeant vers le haut la bordure de la paroi de fond, un film thermoscellable 620 venant refermer de manière étanche l'espace délimité par cette barquette 610. De préférence, l'ensemble 500 est disposé dans l'espace délimité par la barquette 610, l'étiquette 522 étant tournée en direction du film thermoscellable 620 afin que cette étiquette 522 reste visible au travers du film thermoscellable. Ainsi, on comprend que cet ensemble de protection 600 est caractérisé en ce qu'il comporte un ensemble d'emballage et de présentation 500, une barquette de protection 610 formant un contenant pour ledit ensemble d'emballage et de présentation 500 et un film thermoscellable 620 apte à être retiré manuellement refermant ladite barquette 610 de manière étanche.
- un étui de protection et de conditionnement 630 extérieur entoure éventuellement ladite barquette (voir figure 15), cet étui étant réalisé également en matière plastique de préférence transparente ou translucide, cet étui 630 étant également formé d'une partie de fond et d'une partie de couvercle, mobiles entre elles entre une position d'ouverture et une position de fermeture, une étiquette extérieure 640 située de préférence sur la tranche de l'étui 630 permettant de former des moyens de reconnaissance visuelle du type de lot de matériel d'ostéosynthèse qui est contenu dans l'ensemble d'emballage et de présentation 500 logé dans la barquette 610, elle-même placée dans la partie de fond de l'étui 630. A cet effet, un code couleur porté par cette étiquette extérieure 640 pourra désigner un type particulier de lot de matériel d'ostéosynthèse.

Ainsi, on comprend que l'étui de protection et de conditionnement extérieur 630 porte des moyens de reconnaissance visuelle 640 du type de lot de matériel d'ostéosynthèse qui est contenu dans ledit ensemble d'emballage et de présentation 500.

Egalement, on comprend que l'ensemble de protection 600 est caractérisé en ce que ledit étui de protection et de conditionnement extérieur 630 comporte une partie de fond et une partie de couvercle mobiles l'un par rapport à l'autre entre une position d'ouverture et une position de fermeture, et en ce que ledit étui de protection et de conditionnement extérieur 630 comporte en outre des moyens de fermeture réversibles disposés sur ladite partie de fond et sur ladite partie de couvercle qui sont aptes à coopérer dans ladite position de fermeture.

Il faut noter que grâce à l'étiquette 640 située sur chacun de ces étuis de protection et de conditionnement 630 formant l'enveloppe extérieure des ensembles de protection 600, il est possible de connaître rapidement et simplement le type particulier de lot de matériel d'ostéosynthèse contenu dans chaque ensemble de protection 600, afin de choisir l'étui contenant l'ensemble d'emballage et de présentation 500 qui aura été pourvu du lot de matériel d'ostéosynthèse qui est recherché.

Il est à noter que les éléments formant l'ensemble d'ostéosynthèse montré en relation avec le mode de réalisation décrit précédemment, ne doivent pas se limiter à du matériel pour réaliser une ostéotomie, notamment de la mandibule, mais qu'il convient de considérer également d'autres interventions de chirurgie maxillo-faciale, ou plus généralement toute intervention chirurgicale d'ostéosynthèse.

Ainsi, de manière plus générale, le lot de matériel d'ostéosynthèse peut comprendre une (des) plaque(s) et/ou une (des) vis et/ou une (des) broches et/ou un (des) fil(s) métallique(s) .....

## Revendications

1. Emballage (100) pour la présentation individuelle d'au moins une vis (110) comprenant une tige et une tête, en particulier pour une vis (110) d'ostéosynthèse, ledit emballage (100) comportant un tube (102) apte à être saisi manuellement et présentant une première extrémité (102a) et une deuxième extrémité (102b), ledit tube (102) étant pourvu d'au moins un logement (106) destiné à recevoir de manière unitaire ladite vis (110), ledit logement (106) présentant une ouverture (106a) débouchant sur ladite première extrémité (102a), et des moyens de fermeture (104 ;116) refermant ledit logement (106) de manière étanche et aptes à être ouverts, ledit logement (106) s'étendant selon une direction longitudinale et présentant une première portion (106b) destinée à recevoir ladite tige de la vis (110) et une deuxième portion (106c), ladite deuxième portion (106c) étant plus large que la première portion (106b) et étant apte à recevoir avec appui ladite tête de la vis (110), ladite deuxième extrémité (102b) servant de socle apte à venir en appui sur un plan sensiblement horizontal, en particulier afin de permettre d'accéder à la vis (110) située dans ledit logement (106) depuis ladite ouverture (106a) lorsque lesdits moyens de fermeture (104 ;116) sont ouverts, ledit socle étant perpendiculaire à ladite direction longitudinale, ledit emballage (100) étant **caractérisé en ce que** ledit tube (102) est creux et **en ce que** ledit logement (106) est formé dans une pièce tubulaire (114) disposée au moins partiellement à l'intérieur dudit tube (102), un espace fermé étant formé entre ledit tube (102) et ladite pièce tubulaire (114).

2. Emballage (100) selon la revendication 1, **caractérisé en ce que** ledit tube (102) est cylindrique de section transversale au moins partiellement circulaire.

3. Emballage (100) selon l'une quelconque des revendications précédentes, **caractérisé en ce que** lesdits moyens de fermeture comprennent un bouchon (104) et **en ce que** ledit logement (106) présente une troisième portion (106d) plus large que la deuxième portion (106c) qui lui est adjacente, ladite troisième portion (106d) étant apte à recevoir ledit bouchon (104) de manière serrée de manière à refermer ledit logement (106) de manière réversible.

4. Emballage (100) selon l'une quelconque des revendications précédentes, **caractérisé en ce que** lesdits moyens de fermeture comprennent un opercule (116) perforable qui recouvre ladite ouverture (106a).

5. Emballage (100) selon l'une quelconque des revendications précédentes, **caractérisé en ce que** ladite deuxième portion (106c) présente au moins une première partie en forme de tronc de cône allant en s'évasant depuis la première portion (106b) vers ladite ouverture (106a), ladite tête de la vis (110) venant en appui sur ladite forme de tronc de cône.

6. Emballage (100) selon l'une quelconque des revendications précédentes, **caractérisé en ce qu'**entre ladite deuxième portion (106c) et ladite première portion (106b) est formé un épaulement (106e) muni d'une face d'appui (106f) dirigée en direction de ladite ouverture (106a), ladite tête de la vis (110) venant en appui sur ladite face d'appui (106f) sensiblement plane.

7. Emballage (100) selon l'une quelconque des revendications précédentes, **caractérisé en ce que** le tube (102) comporte un second logement (108) destiné à recevoir de manière unitaire une seconde vis (112), ledit second logement (108) présentant une ouverture (108a) débouchant sur ladite deuxième extrémité (102b), et des seconds moyens de fermeture (104 ;116) refermant ledit second logement (108) de manière étanche et aptes à être ouvert, ledit second logement (108) s'étendant selon une direction longitudinale et présentant une première portion (108b) destinée à recevoir ladite tige de la seconde vis (112) et une deuxième portion (108c), ladite deuxième portion (108c) étant plus large que la première portion (108b) et étant apte à recevoir avec appui ladite tête de la seconde vis (112), ladite première extrémité (102a) servant de socle apte à venir en appui sur un plan sensiblement horizontal, en particulier afin de permettre d'accéder à la vis (112) située dans ledit second logement (108) depuis ladite ouverture (108a) dudit second logement (108) lorsque lesdits seconds moyens de fermeture (104 ;116) sont ouverts, ledit socle étant perpendiculaire à ladite direction longitudinale.

8. Emballage (100) selon l'une quelconque des revendications précédentes, **caractérisé en ce que** ledit tube (102) porte au moins un support d'identification (122) portant des informations visuelles en relation avec ladite vis (110), lesdites informations visuelles comportant un code à barres.

9. Ensemble d'emballage (200) et de présentation individuelle d'une série de vis (110) comprenant une tige et une tête, en particulier pour des vis (110) d'ostéosynthèse, **caractérisé en ce qu'**il comporte au moins deux emballages (100) selon l'une quelconque des revendications 1 à 8 renfermant chacun ladite vis (110) dans le logement (106) et une enveloppe (202) permettant de relier entre eux lesdits emballages (100) en les retenant un à un de manière étanche sous forme d'un chapelet.

10. Ensemble (300) d'emballage et de présentation individuelle d'une vis (110) comprenant une tige et une tête, en particulier une vis (110) d'ostéosynthèse, **caractérisé en ce qu'**il comporte un étui de protection et de conditionnement (302) entourant un emballage (100) selon l'une quelconque des revendications 1 à 8, ledit emballage (100) renfermant ladite vis (110) dans le logement (106).

11. Ensemble (300) selon la revendication 10, **caractérisé en ce que** ledit étui de protection et de conditionnement (302) est réalisé en matière plastique sous forme d'une partie rigide (304) délimitant un espace (306) renfermant ledit emballage (100) et d'une partie souple formée d'un film (308) thermoscellable refermant de manière étanche ledit espace (306) et apte à être retiré manuellement.

12. Ensemble (400) d'emballage et de présentation d'un lot de matériel d'ostéosynthèse, ledit lot de matériel d'ostéosynthèse comprenant au moins une plaque et au moins une vis (110) d'ostéosynthèse, **caractérisé en ce qu'**il comprend une barquette (402), un film thermoscellable (408) refermant ladite barquette (402) et délimitant avec cette dernière un espace de rangement (406), ladite plaque étant disposée dans ledit espace de rangement, et un emballage (100) selon l'une quelconque des revendications 1 à 8 renfermant ladite vis (110) dans le logement (106), ledit emballage (100) étant disposé dans ledit espace de rangement (406).

13. Ensemble (400) selon la revendication 12, **caractérisé en ce qu'**il comporte en outre au moins un insert (410) placé dans ledit espace de rangement (406) et muni d'au moins une cavité (412) destinée à recevoir au moins une plaque d'ostéosynthèse

14. Ensemble selon la revendication 13, **caractérisé en ce que** ladite cavité comporte des moyens de retenue (414) permettant le positionnement de ladite plaque.

15. Ensemble selon la revendication 13 ou 14, **caractérisé en ce que** ledit insert (412) est en outre équipé d'au moins un alvéole (416) destiné à recevoir au moins une vis (110) d'ostéosynthèse.

16. Ensemble (500) d'emballage et de présentation d'un lot de matériel d'ostéosynthèse, ledit lot de matériel d'ostéosynthèse comprenant au moins une plaque et au moins une vis (110) d'ostéosynthèse, **caractérisé en ce qu'**il comprend un support rigide (502) délimitant plusieurs compartiments de rangement parmi lesquels au moins un compartiment de rangement d'un premier type (504) dans lequel est inséré un emballage (100) selon l'une quelconque des revendications 1 à 8 contenant ladite vis (110) et au moins un compartiment de rangement d'un deuxième type (506) destiné à recevoir au moins une plaque d'ostéosynthèse (130).

17. Ensemble (500) selon la revendication 16, **caractérisé en ce que** lesdits compartiments de rangement comportent en outre au moins un compartiment de rangement d'un troisième type (508) dans lequel est logé un outil d'ostéosynthèse (120).

18. Ensemble (500) selon l'une quelconque des revendications 16 à 17, **caractérisé en ce que** ledit support rigide (502) comprend au moins deux compartiments de rangement du premier type (504) recevant chacun un emballage (100) selon l'une quelconque des revendications 1 à 8 contenant chacun une vis (110).

19. Ensemble (500) selon l'une quelconque des revendications 16 à 18, **caractérisé en ce que** ledit support rigide (502) comprend en outre au moins un compartiment de rangement d'un troisième type (508), et au moins un foret et/ou une lame de tournevis (120), adapté(e)(s) à la dite vis (110) et disposé(e)(s) dans ledit compartiment de rangement du troisième type (508) du support rigide (502).

20. Ensemble (500) selon l'une quelconque des revendications 16 à 19, **caractérisé en ce que** ledit support rigide (502) comprend en outre au moins un compartiment de rangement d'un quatrième type (510), et au moins un tournevis (120) disposé dans ledit compartiment de rangement du quatrième type (510) du support rigide (502).

21. Ensemble (500) selon la revendication 20, **caractérisé en ce que** ledit tournevis (120) comporte une partie de préhension munie d'un tronçon arrière destiné à venir se placer dans le creux de la main et d'un tronçon avant monté rotatif par rapport au tronçon arrière et apte à porter la lame du tournevis (120).

22. Ensemble (500) d'emballage et de présentation selon l'une quelconque des revendications 16 à 21, **caractérisé en ce que** ledit support rigide (502) forme une partie de fond et **en ce qu'**il comprend en outre une partie de couvercle (520), ladite partie de couvercle (520) étant apte à coopérer de manière réversible avec ladite partie de fond (502) entre une position d'ouverture et une position de fermeture, et **en ce que**, dans ladite position de fermeture, ladite partie de couvercle (520) est apte à retenir ledit emballage (100) dans ledit compartiment de rangement du premier type (504) et ladite plaque d'ostéosynthèse (130) dans ledit compartiment de rangement du deuxième type (506).

23. Ensemble (500) d'emballage et de présentation selon la revendication 22, **caractérisé en ce que** ladite partie de couvercle (520) présente, sur sa face opposée à ladite partie de fond, un espace de rangement (532) contenant une plaque fantôme et refermé de manière étanche par un film thermoscellable (534) apte à être retiré manuellement.

24. Ensemble (200 ; 300 ; 400 ; 500) d'emballage et de présentation selon l'une quelconque des revendications 9 à 23, **caractérisé en ce qu'**il comprend en outre un support d'identification (522) portant des informations visuelles en relation avec ladite vis (110) d'ostéosynthèse, lesdites informations visuelles comportant un code à barres.

25. Ensemble de protection (600), **caractérisé en ce qu'**il comporte un ensemble (200 ; 300 ; 400 ; 500) d'emballage et de présentation selon l'une quelconque des revendications 9 à 24, une barquette de protection (610) formant un contenant pour ledit ensemble (200 ; 300 ; 400 ; 500) d'emballage et de présentation et un film thermoscellable (620) apte à être retiré manuellement refermant ladite barquette de protection (610) de manière étanche.

26. Ensemble de protection (600) selon la revendication 25, **caractérisé en ce qu'**il comprend en outre un étui de protection et de conditionnement extérieur (630) entourant ladite barquette (610).

27. Ensemble de protection (600) selon la revendication 26, **caractérisé en ce que** ledit étui de protection et de conditionnement extérieur (630) porte des moyens de reconnaissance visuelle (640) du type de matériel d'ostéosynthèse qui est contenu dans ledit ensemble (200 ; 300 ; 400 ; 500) d'emballage et de présentation.

28. Ensemble de protection (600) selon l'une quelconque des revendications 26 et 27, **caractérisé en ce que** ledit étui de protection et de conditionnement extérieur (630) comporte une partie de fond et une partie de couvercle mobiles l'un par rapport à l'autre entre une position d'ouverture et une position de fermeture, et **en ce que** ledit étui de protection et de conditionnement extérieur (630) comporte en outre des moyens de fermeture réversible disposés sur ladite partie de fond et sur ladite partie de couvercle qui sont aptes à coopérer dans ladite position de fermeture.

## Patentansprüche

1. Verpackung (100) zur individuellen Bereitstellung mindestens einer Schraube (110), umfassend einen Schaft und einen Kopf, insbesondere für eine Osteosynthese-Schraube (110), wobei die Verpackung (100) eine Röhre (102) umfasst, die geeignet ist, manuell erfasst zu werden, und ein erstes Ende (102a) und ein zweites Ende (102b) aufweist, wobei die Röhre (102) mit mindestens einer Aufnahme (106) versehen ist, die dazu bestimmt ist, die Schraube (110) einzeln aufzunehmen, wobei die Aufnahme (106) eine Öffnung (106a) aufweist, die an dem ersten Ende (102a) mündet, und Verschlussmittel (104; 116), die die Aufnahme (106) dicht verschließen und geeignet sind, geöffnet zu werden, wobei sich die Aufnahme (106) in eine Längsrichtung erstreckt und einen ersten Abschnitt (106b), der dazu bestimmt ist, den Schaft der Schraube (110) aufzunehmen, und einen zweiten Abschnitt (106c) aufweist, wobei der zweite Abschnitt (106c) breiter als der erste Abschnitt (106b) ist und geeignet ist, unter Abstützung den Kopf der Schraube (110) aufzunehmen, wobei das zweite Ende (102b) als Sockel dient, der geeignet ist, auf einer im Wesentlichen horizontalen Ebene zur Auflage zu gelangen, um insbesondere den Zugang zu der Schraube (110), die sich in der Aufnahme (106) befindet, von der Öffnung (106a) aus zu ermöglichen, wenn die Verschlussmittel (104; 116) offen sind, wobei der Sockel auf die Längsrichtung senkrecht ist, wobei die Verpackung (100) **dadurch gekennzeichnet ist, dass** die Röhre (102) hohl ist, und dass die Aufnahme (106) in einem röhrenförmigen Teil (114) ausgebildet ist, das zumindest teilweise innerhalb der Röhre (102) angeordnet ist, wobei ein geschlossener Raum zwischen der Röhre (102) und dem röhrenförmigen Teil (114) ausgebildet ist.

2. Verpackung (100) nach Anspruch 1, **dadurch gekennzeichnet, dass** die Röhre (102) zylindrisch mit einem zumindest teilweise kreisförmigen Querschnitt ist.

3. Verpackung (100) nach einem der vorhergehenden Ansprüche, **dadurch gekennzeichnet, dass** die Verschlussmittel einen Stöpsel (104) umfassen, und dass die Aufnahme (106) einen dritten (106d) breiteren Abschnitt als der zweite Abschnitt (106c), der an diesen grenzt, aufweist, wobei der dritte Abschnitt (106d) geeignet ist, den Stöpsel (104) fest aufzunehmen, um die Aufnahme (106) auf reversible Weise zu schließen.

4. Verpackung (100) nach einem der vorhergehenden Ansprüche, **dadurch gekennzeichnet, dass** die Verschlussmittel einen perforierbaren Deckel (116) umfassen, der die Öffnung (106a) bedeckt.

5. Verpackung (100) nach einem der vorhergehenden Ansprüche, **dadurch gekennzeichnet, dass** der zweite Abschnitt (106c) zumindest einen ersten kegelstumpfartigen Teil aufweist, der sich vom ersten Abschnitt (106b) zur Öffnung (106a) hin erweitert, wobei der Kopf der Schraube (110) an der Kegelstumpfform zur Anlage gelangt.

6. Verpackung (100) nach einem der vorhergehenden Ansprüche, **dadurch gekennzeichnet, dass** zwischen dem zweiten Abschnitt (106c) und dem ersten Abschnitt (106b) ein Absatz (106e) ausgebildet ist, der mit einer Stützfläche (106f) versehen ist, die in Richtung der Öffnung (106a) gerichtet ist, wobei der Kopf der Schraube (110), der auf der Stützfläche (106f) zur Auflage gelangt, im Wesentlichen flach ist.

7. Verpackung (100) nach einem der vorhergehenden Ansprüche, **dadurch gekennzeichnet, dass** die Röhre (102) eine zweite Aufnahme (108) umfasst, die dazu bestimmt ist, einzeln eine zweite Schraube (112) aufzunehmen, wobei die zweite Aufnahme (108) eine Öffnung (108a), die an dem zweiten Ende (102b) mündet, und zweite Verschlussmittel (104; 116) aufweist, die die zweite Aufnahme (108) dicht verschließen und geeignet sind, geöffnet zu werden, wobei sich die zweite Aufnahme (108) in Längsrichtung erstreckt und einen ersten Abschnitt (108b), der dazu bestimmt ist, den Schaft der zweiten Schraube (112) aufzunehmen, und einen zweiten Abschnitt (108c) umfasst, wobei der zweite Abschnitt (108c) breiter als der erste Abschnitt (108b) ist und geeignet ist, unter Abstützung den Kopf der zweiten Schraube (112) aufzunehmen, wobei das erste Ende (102a) als Sockel dient, der geeignet ist, auf einer im Wesentlichen horizontalen Ebene zur Auflage zu gelangen, um insbesondere den Zugang zu der Schraube (112), die sich in der zweiten Aufnahme (108) befindet, von der Öffnung (108a) der zweiten Aufnahme (108) aus zu ermöglichen, wenn die zweiten Verschlussmittel (104; 116) offen sind, wobei der Sockel auf die Längsrichtung senkrecht ist.

8. Verpackung (100) nach einem der vorhergehenden Ansprüche, **dadurch gekennzeichnet, dass** die Röhre (102) mindestens einen Identifikationsträger (122) trägt, der visuelle Informationen in Verbindung mit der Schraube (110) umfasst, wobei die visuellen Informationen einen Barcode haben.

9. Einheit (200) zur Verpackung und individuellen Bereitstellung einer Reihe von Schrauben (110), umfassend einen Schaft und einen Kopf, insbesondere für Osteosynthese-Schrauben (110), **dadurch gekennzeichnet, dass** sie mindestens zwei Verpackungen (100) nach einem der Ansprüche 1 bis 8 umfasst, die jeweils die Schraube (110) in der Aufnahme (106) einschließen, und eine Hülle (202) umfasst, die es ermöglicht, die Verpackungen (100) miteinander zu verbinden, wobei sie sie einzeln auf dichte Weise in Form einer Kette hält.

10. Einheit (300) zur Verpackung und individuellen Bereitstellung einer Schraube (110), umfassend einen Schaft und einen Kopf, insbesondere einer Osteosynthese-Schraube (110), **dadurch gekennzeichnet, dass** sie eine Schutz- und Verpackungshülle (302) umfasst, die eine Verpackung (100) nach einem der Ansprüche 1 bis 8 umgibt, wobei die Verpackung (100) die Schraube (110) in der Aufnahme (106) einschließt.

11. Einheit (300) nach Anspruch 10, **dadurch gekennzeichnet, dass** die Schutz- und Verpackungshülle (302) aus Kunststoff in Form eines starren Teils (304), der einen Raum (306), der die Verpackung (100) einschließt, begrenzt, und eines biegsamen Teils, der von einer Heißsiegelfolie (308) gebildet ist, die den Raum (306) dicht verschließt und manuell abgezogen werden kann.

12. Einheit (400) zur Verpackung und Bereitstellung eines Osteosynthese-Materialpakets, wobei das Osteosynthese-Materialpaket mindestens eine Platte und mindestens eine Osteosynthese-Schraube (110) umfasst, **dadurch gekennzeichnet, dass** sie eine Schale (402) umfasst, wobei eine Heißsiegelfolie (408) die Schale (402) verschließt und mit dieser letztgenannten einen Stauraum (406) begrenzt, wobei die Platte in dem Stauraum angeordnet ist, und eine Verpackung (100) nach einem der Ansprüche 1 bis 8 umfasst, die die Schraube (110) in der Aufnahme (106) einschließt, wobei die Verpackung (100) in dem Stauraum (406) angeordnet ist.

13. Einheit (400) nach Anspruch 12, **dadurch gekennzeichnet, dass** sie ferner mindestens einen Einsatz (410) umfasst, der in dem Stauraum (406) angeordnet ist und mit mindestens einem Hohlraum (412) versehen ist, der dazu bestimmt ist, mindestens eine Osteosynthese-Platte aufzunehmen.

14. Einheit nach Anspruch 13, **dadurch gekennzeichnet, dass** der Hohlraum Haltemittel (414) umfasst, die die Positionierung der Platte ermöglichen.

15. Einheit nach Anspruch 13 oder 14, **dadurch gekennzeichnet, dass** der Einsatz (412) ferner mit mindestens einem Fach (416) versehen ist, das dazu bestimmt ist, zumindest eine Osteosynthese-Schraube (110) aufzunehmen.

16. Einheit (500) zur Verpackung und Bereitstellung eines Osteosynthese-Materialpakets, wobei das Osteosynthese-Materialpaket mindestens eine Platte und mindestens eine Osteosynthese-Schraube (110) umfasst, **dadurch gekennzeichnet, dass** sie einen starren Träger (502) umfasst, der mehrere Staufächer begrenzt, unter anderem mindestens ein Staufach eines ersten Typs (504), in dem eine Verpackung (100) nach einem der Ansprüche 1 bis 8, die die Schraube (110) enthält, untergebracht ist, und mindestens ein Staufach eines zweiten Typs (506), das dazu bestimmt ist, mindestens eine Osteosynthese-Platte (130) aufzunehmen.

17. Einheit (500) nach Anspruch 16, **dadurch gekennzeichnet, dass** die Staufächer ferner mindestens ein Staufach eines dritten Typs (508) umfassen, in dem ein Osteosynthese-Werkzeug (120) angeordnet ist.

18. Einheit (500) nach einem der Ansprüche 16 bis 17, **dadurch gekennzeichnet, dass** der starre Träger (502) mindestens zwei Staufächer des ersten Typs (504) umfasst, die jeweils eine Verpackung (100) nach einem der Ansprüche 1 bis 8, die jeweils eine Schraube (110) enthält, aufnehmen.

19. Einheit (500) nach einem der Ansprüche 16 bis 18, **dadurch gekennzeichnet, dass** der starre Träger (502) ferner mindestens ein Staufach eines dritten Typs (508) und mindestens einen Bohrer und/oder eine Schraubenzieherklinge (120) umfasst, die an die Schraube (110) angepasst und in dem Staufach des dritten Typs (508) des starren Trägers (502) angeordnet sind.

20. Einheit (500) nach einem der Ansprüche 16 bis 19, **dadurch gekennzeichnet, dass** der starre Träger (502) ferner mindestens ein Staufach eines vierten Typs (510) und mindestens einen Schraubenzieher (120) umfasst, der in dem Staufach des vierten Typs (510) des starren Trägers (502) angeordnet ist.

21. Einheit (500) nach Anspruch 20, **dadurch gekennzeichnet, dass** der Schraubenzieher (120) einen Griff umfasst, der mit einem hinteren Abschnitt, der dazu bestimmt ist, in dem Hohlraum der Hand angeordnet zu sein, und einem vorderen Abschnitt versehen ist, der drehbar in Bezug zum hinteren Abschnitt montiert und geeignet ist, die Klinge des Schraubenziehers (120) zu tragen.

22. Einheit (500) zur Verpackung und Bereitstellung nach einem der Ansprüche 16 bis 21, **dadurch gekennzeichnet, dass** der starre Träger (502) einen Bodenteil bildet, und dass er ferner einen Deckelteil (520) umfasst, wobei der Deckelteil (520) geeignet ist, auf reversible Weise mit dem Bodenteil (502) zwischen einer Öffnungsposition und einer Verschlussposition zusammenzuwirken, und dass in der Verschlussposition der Deckelteil (520) geeignet ist, die Verpackung (100) in dem Staufach des ersten Typs (504) und die Osteosynthese-Platte (130) in dem Staufach des zweiten Typs (506) zu halten.

23. Einheit (500) zur Verpackung und Bereitstellung nach Anspruch 22, **dadurch gekennzeichnet, dass** der Deckelteil (520) auf seiner dem Bodenteil gegenüberliegenden Seite einen Stauraum (532) aufweist, der eine Phantomplatte enthält und dicht durch eine Heißsiegelfolie (534), die geeignet ist, manuell abgezogen zu werden, verschlossen ist.

24. Einheit (200; 300; 400; 500) zur Verpackung und Bereitstellung nach einem der Ansprüche 9 bis 23, **dadurch gekennzeichnet, dass** sie ferner einen Identifikationsträger (522) umfasst, der visuelle Informationen in Verbindung mit der Osteosynthese-Schraube (110) trägt, wobei die visuellen Informationen einen Barcode umfassen.

25. Schutzeinheit (600), **dadurch gekennzeichnet, dass** sie eine Einheit (200; 300; 400; 500) zur Verpackung und Bereitstellung nach einem der Ansprüche 9 bis 24, wobei eine Schutzschale (610) ein Behältnis für die Einheit (200; 300; 400; 500) zur Verpackung und Bereitstellung bildet, und eine Heißsiegelfolie (620) umfasst, die geeignet ist, manuell abgezogen zu werden, die die Schutzschale (610) dicht verschließt.

26. Schutzeinheit (600) nach Anspruch 25, **dadurch gekennzeichnet, dass** sie ferner eine äußere Schutz- und Verpackungshülle (630) umfasst, die die Schale (610) umgibt.

27. Schutzeinheit (600) nach Anspruch 26, **dadurch gekennzeichnet, dass** die äußere Schutz- und Verpackungshülle (630) visuelle Erkennungsmittel (640) des Osteosynthese-Materialtyps, der in der Einheit (200; 300; 400; 500) zur Verpackung und Bereitstellung enthalten ist, trägt.

28. Schutzeinheit (600) nach einem der Ansprüche 26 und 27, **dadurch gekennzeichnet, dass** die äußere Schutz- und Verpackungshülle (630) einen Bodenteil und einen Deckelteil umfasst, die in Bezug zueinander zwischen einer Öffnungsposition und einer Verschlussposition beweglich sind, und dass die äußere Schutz- und Verpackungshülle (630) ferner reversible Verschlussmittel umfasst, die auf dem Bodenteil und auf dem Deckelteil angeordnet und geeignet sind, in Verschlussposition zusammenzuwirken.

## Claims

1. Packaging (100) for individually presenting at least one screw (110) comprising a shank and a head, in particular for an osteosynthesis screw (110), wherein the packaging (100) comprises a tube (102) suitable for being held in the hand and presenting a first end (102a) and a second end (102b), said tube (102) being provided with at least one housing (106) for receiving said screw (110) in unitary manner, said housing (106) presenting an opening (106a) into said first end (102a), and closure means (104; 116) closing said housing (106) in sealed manner and suitable for being opened, said housing (106) extending in a longitudinal direction and presenting a first portion (106b) for receiving said shank of the screw (110) and a second portion (106c), said second portion (106c) being larger than the first portion (106b) and being suitable for receiving and supporting said head of the screw (110), said second end (102b) serving as a stand suitable for supporting on a substantially horizontal plane, in particular in order to give access to the screw (110) situated in said housing (106) via said opening (106a) when said closure means (104, 116) are open, with said stand extending perpendicularly to said longitudinal direction, said packaging being **characterized in that** said tube (102) is hollow and **in that** said housing (106) is formed in a tubular part (114) disposed at least in part inside said tube (102), a closed space being formed between said tube (102) and said tubular part (114).

2. Packaging (100) according to claim 1, **characterized in that** said tube (102) is cylindrical and of cross-section that is at least partially circular.

3. Packaging (100) according to any one of the previous claims, **characterized in that** said closure means comprise a cap (104), and **in that** said housing (106) presents a third portion (106d) larger than the second portion (106c) that is adjacent thereto, said third portion (106d) being suitable for receiving said cap tightly so as to close said housing (106) in reversible manner.

4. Packaging (100) according to any one of the previous claims, **characterized in that** said closure means comprise a perforatable membrane seal (116) covering said opening (106a).

5. Packaging (100) according to any one of the previous claims, **characterized in that** said second portion (106c) presents at least one first part in the form of a truncated cone flaring from the first portion (106b) towards said opening (106a), said head of the screw (110) bearing against said truncated cone shape.

6. Packaging (100) according to any one of the previous claims, **characterized in that** between said second portion (106c) and said first portion (106b), there is formed a shoulder (106e) provided with a bearing face (106f) facing towards said opening (106a), said head of the screw (110) bearing against said face (106f) substantially plane.

7. Packaging (100) according to any one of the previous claims, **characterized in that** the tube (102) has a second housing (108) for receiving a second screw (112) in unitary manner, said second housing (108) presenting an opening (108a) opening out into said second end (102b), and second closure means (104;116) closing said second housing (108) in sealed manner and suitable for being opened, said second housing (108) extending in a longitudinal direction and presenting a first portion (108b) for receiving said shank of the second screw (112) and a second portion (108c), said second portion (108c) being larger than the first portion (108b) and being suitable for receiving and supporting said head of the second screw (112), said first end (102a) serving as a stand suitable for supporting on a substantially horizontal plane, in particular in order to allow access to the screw (112) situated in said second housing (108) via said opening (108a) of said second housing (108) when said closure means (104;116) are open, said stand being perpendicular to said longitudinal direction.

8. Packaging (100) according to any one of the previous claims, **characterized in that** said tube (102) carries at least one identification medium (122) carrying visible information relating to said screw (110), said visible information comprising a bar code.

9. Assembly for packaging (200) and presenting individually a series of screws (110) comprising a shank and a head, in particular for osteosynthesis screws (110), **characterized in that** the assembly includes at least two packagings (100) according to any one of claims 1 to 8 containing each said screw (110) in the housing (106), and an envelope (202) enabling said packagings (100) to be interconnected by holding them one by one in sealed manner in the form of a string.

10. Assembly (300) for packaging and presenting individually a screw (110) comprising a shank and a head, in particular an osteosynthesis screw (110), **characterized in that** the assembly comprises a protective and packaging case (302) surrounding a packaging (100) according to any one of claims 1 to 8, said packaging (100) containing said screw (110) in the housing (106).

11. Assembly (300) according to claim 10, **characterized in that** said protective and packaging case (302) is made of plastics material in the form a rigid portion (304) defining a space (306) containing said packaging (100) and a flexible portion formed by a heat-sealable film (308) closing said space (306) in sealed manner and suitable for being withdrawn by hand.

12. Assembly (400) for packaging and presenting an osteosynthesis equipment set, said osteosynthesis equipment set comprising at least one osteosynthesis screw (110) and at least one plate, **characterized in that** the assembly comprises a tray (402), a heat-sealable film (408) closing said tray (402) and defining, with the tray, a storage space (406), said plate being disposed in said storage space, and a packaging (100) according to any one of claims 1 to 8 containing said screw (110) in the housing (106), said packaging (100) being disposed in said storage space (406).

13. Assembly (400) according to claim 12, **characterized in that** it further comprises at least one insert (410) placed in said storage space (406) and provided with at least one cavity (412) for receiving at least one osteosynthesis plate.

14. Assembly according to claim 13, **characterized in that** said cavity includes retaining means (414) enabling said plate to be positioned.

15. Assembly according to claim 13 or 14, **characterized in that** said insert (412) is further fitted with at least one cell (416) for receiving at least one osteosynthesis screw (110).

16. Assembly (500) for packaging and presenting an osteosynthesis equipment set, said osteosynthesis equipment set comprising at least one osteosynthesis screw (110) and at least one plate, **characterized in that** the assembly comprises a rigid support (502) defining a plurality of storage compartments including at least one storage compartment of a first type (504) into which a packaging (100) according to any one of claims 1 to 8 containing said screw (110) is inserted, and at least one storage compartment of a second type (506) for receiving at least one osteosynthesis plate (130).

17. Assembly (500) according to claim 16, **characterized in that** said storage compartments further comprise at least one storage compartment of a third type (508) in which there is housed an osteosynthesis tool (120).

18. Assembly according to any one of claims 16 to 17, **characterized in that** said rigid support (502) includes at least two storage compartments of the first type (504) each receiving a packaging (100) according to any one of claims 1 to 8 containing each a screw (110).

19. Assembly according to any one of claims 16 to 18, **characterized in that** said rigid support (502) further includes at least one storage compartment of a third type (508), and at least one drill bit and/or screwdriver blade (120) matching said screw (110) and placed in said storage compartment of the third type (508) of the rigid support (502).

20. Assembly (500) according to any one of claims 16 to 19, **characterized in that** said rigid support (502) further includes at least one storage compartment of a fourth type (510), and at least one screwdriver (120) disposed in said storage compartment of the fourth type (510) of the rigid support (502).

21. Assembly (500) according to claim 20, **characterized in that** said screwdriver (120) comprises a handle portion provided with a rear segment for being placed in the hollow of the hand, and a front segment mounted to turn relative to the rear segment and suitable for carrying the screwdriver blade (120).

22. Assembly (500) for packaging and presenting according to any one of claims 16 to 21, **characterized in that** said rigid portion (502) forms a bottom portion, and **in that** the assembly further includes a lid portion (520), said lid portion (520) being suitable for co-operating in reversible manner with said bottom portion (502) between an open position and a closed position, and **in that** in said closed position, said lid portion (520) is suitable for holding said packaging (100) in said storage compartment of the first type (504) and said osteosynthesis plate (130) in said storage compartment of the second type (506).

23. Assembly (500) for packaging and presenting according to claim 22, **characterized in that** said lid portion (520) presents in its face opposed to the bottom portion, a storage space (532) containing a dummy plate and being closed in sealed manner by a heat-sealable film (534) suitable for being withdrawn by hand.

24. Assembly (200;300;400;500) for packaging and presenting according to any one of claims 9 to 23, **characterized in that** it further includes an identification medium (522) presenting visible information associated with said osteosynthesis screw (110), said visible information comprising a bar code.

25. Protective assembly (600), **characterized in that** it comprises an assembly (200;300;400;500) for packaging and presenting according to any one of claims 9 to 24, a protective tray (610) forming a container for said assembly (200;300;400;500) for packaging and presenting, and a heat-sealable film (620) suitable for being withdrawn by hand and closing said protective tray (610) in sealed manner.

26. Protective assembly (600) according to claim 25, **characterized in that** it further comprises an outer protective and packaging case (630) surrounding said tray (610).

27. Protective assembly (600) according to claim 26, **characterized in that** said outer protective and packaging case (630) carries visible recognition means (640) for the type of osteosynthesis equipment contained in said assembly (200;300;400;500) for packaging and presenting.

28. Protective assembly (600) according to any one of claims 26 and 27, **characterized in that** said outer protective and packaging case (630) comprises a bottom portion and a lid portion movable relative to each other between an open position and a closed position, and **in that** said outer protective and packaging case (630) further includes reversible closure means disposed on said bottom portion and on said lid portion and suitable for co-operating in said closed position.
